# EUROPEAN PATENT APPLICATION

(11) **EP 3 479 764 A1**
(43) Date of publication of application: **08.05.2019**
(21) Application number: 18201871.3
(22) Date of filing: 22.10.2018
(51) Int. Cl.: A61B 5/024, A61B 5/0245, A61B 5/0404, A61B 5/021, A61B 5/00

(54) **BIOSENSOR CONFIGURATON WHICH CAN DETECT AT LEAST TWO PHYSIOLOGICAL FEATURES SIMULTANEOUSLY WITH ONLY TWO CONTACT POSITIONS IN MOBILE DEVICE**

(30) Priority: 01.11.2017 US 201762579939 P; 03.10.2018 US 201816150282
(71) Applicant: MEDIATEK INC., Hsin-Chu 300 (TW)
(72) Inventor: KUO, Jing-Lin, 30078 Hsinchu (TW); ZOU, Teng-Feng, 30078 Hsinchu (TW); LIN, Chih-Chun, 30078 Hsinchu (TW); CHI, Chia-Wei, 30078 Hsinchu (TW); LIN, Hung-Chih, 30078 Hsinchu (TW); CHEN, Yu-Wen, 30078 Hsinchu (TW)
(74) Representative: Haseltine Lake LLP

(57) **Abstract**

The present invention provides an electronic device including a first electrode set and a second electrode set with an optical sensor. The first electrode set is arranged to contact a first position of a human body, and the second electrode set with the optical sensor is arranged contact a second position of the human body, to receive a plurality of physiological signals to determine at least two physiological features simultaneously.

## Description

This application claims the priority of US Provisional Application No. 62/579,939, filed on November 1th, 2017.

### Background

Recently, personal biosensors become popular for providing physiological information at all time for the reference to the user. However, the current designs of the biosensors may be inconvenient to the user.

### Summary

It is therefore an objective of the present invention to provide a biosensor configuration which can detect at least two physiological features simultaneously upon contacts only with a user's two body positions, to solve the above-mentioned problems.

According to one embodiment of the present invention, an electronic device is includes a first electrode set and a second electrode set with an optical sensor. The first electrode set is arranged to contact a first position of a human body, and the second electrode set with the optical sensor is arranged contact a second position of the human body, to receive a plurality of physiological signals to determine at least two physiological features simultaneously.

According to another embodiment of the present invention, a method for generating at least two cardiac health statues is disclosed, and the method comprises the steps of: using a first electrode set to receive a first physiological signal corresponding a first position of a human body; using a second electrode set to receive a second physiological signal corresponding a second position of the human body; using an optical sensor to receive a third physiological signal; and determining the at least two physiological features according to the first physiological signal, the second physiological signal and the third physiological signal.

These and other objectives of the present invention will no doubt become obvious to those of ordinary skill in the art after reading the following detailed description of the preferred embodiment that is illustrated in the various figures and drawings.

### Brief Description of the Drawings

FIG. 1 is a diagram illustrating an electronic device according to a first embodiment of the present invention.
FIG. 2 shows how to use the electronic device shown in FIG. 1 according one embodiment of the present invention.
FIG. 3 is a diagram illustrating an electronic device according to a second embodiment of the present invention.
FIG. 4 shows how to use the electronic device shown in FIG. 3 according one embodiment of the present invention.
FIG. 5 is a diagram illustrating an electronic device according to a third embodiment of the present invention.
FIG. 6 shows how to use the electronic device shown in FIG. 5 according one embodiment of the present invention.
FIG. 7 is a diagram illustrating an electronic device according to a fourth embodiment of the present invention.
FIG. 8 is a diagram illustrating an electronic device according to a fifth embodiment of the present invention.
FIG. 9 shows how to use the electronic device shown in FIG. 8 according one embodiment of the present invention.

### Detailed Description

Certain terms are used throughout the following description and claims to refer to particular system components. As one skilled in the art will appreciate, manufacturers may refer to a component by different names. This document does not intend to distinguish between components that differ in name but not function. In the following discussion and in the claims, the terms "including" and "comprising" are used in an open-ended fashion, and thus should be interpreted to mean "including, but not limited to ...". The terms "couple" and "couples" are intended to mean either an indirect or a direct electrical connection. Thus, if a first device couples to a second device, that connection may be through a direct electrical connection, or through an indirect electrical connection via other devices and connections.

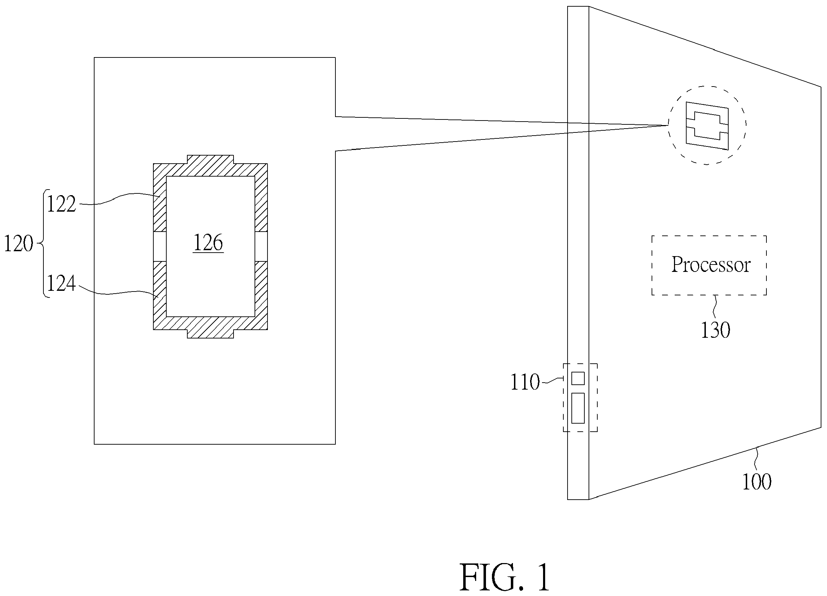

FIG. 1 is a diagram illustrating an electronic device 100 according to a first embodiment of the present invention. The electronic device 100 comprises a first electrode set 110, a second electrode set 120 with an optical sensor 126 and a processor 130. In this embodiment, the first electrode set 110 comprises two electrodes, the second electrode set 120 comprises two electrodes 122 and 124, where each of the electrodes 122 and 124 is U-shaped, and the optical sensor 126 is within the second electrodes 122 and 124. In the embodiment shown in FIG. 1, the electronic device 100 is a smartphone, the first electrode set 110 is positioned at a side of the smartphone, and the second electrode set 120 with the optical sensor 126 is positioned at a back case of the smartphone.

It is noted that the shape of the electrodes 122 and 124 shown in FIG. 1 is not a limitation of the present invention. As long as the electrodes 122 and 124 are at a peripheral zone of the optical sensor 126, the electrodes 122 and 124 may have any other shape, e.g., an 0-shaped electrode.

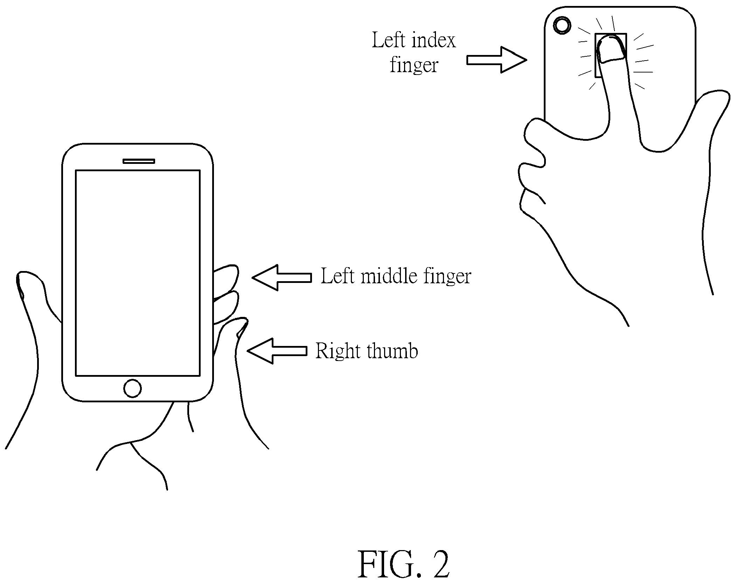

In the operations of the electronic device 100, the first electrode set 110 is arranged to contact a first position of a human body (e.g. right half body or right hand), and the second electrode set 120 with the optical sensor 126 is arranged to contact a second position of the human body (e.g. left half body or left hand), to receive a plurality of physiological signals to determine at least two physiological features simultaneously. Taking FIG. 2 as an example, a user may put a left index finger on the second electrode set 120 with the optical sensor 126, and put a right thumb on the first electrode set 110, where the right hand holds on the left hand to avoid flexing the muscles. In the embodiment shown in FIG. 1 and FIG. 2, the first electrode set 110 and the electrodes 122 and 124 are arranged to provide physiological signals, and the processor 130 generates an electrocardiography (ECG) signal (e.g. lead I ECG signal) according to the physiological signals from the first electrode set 110 and the electrodes 122 and 124; and the optical sensor 126 may comprise at least one light emitter and a photodiode, the light emitter generates light (e.g. visible or infrared light) to the left index finger of the user, and the photodiode receives the reflected light to generate a physiological signal, and the processor 130 generates an photoplethysmography (PPG) signal according to the physiological signal from the optical sensor 126. By analyzing the ECG signal and the PPG signal, e.g. analyzing the waveforms and periods of the ECG signal and the PPG signal, the at least two physiological features such as cardiac health statues can be obtained simultaneously, and the physiological features can be displayed on a screen of the electronic device 100 for the reference to the user. For example, the at least two physiological features may comprise cardiac health statues like ECG, heart rate, blood oxygen saturation (Sp02), blood pressure, or other information like bio-impedance, body fat, body muscle, temperature, blood content analysis, or tissue content concentrations (St02, collagen, melanin, water...etc).

In the embodiment shown in FIG. 1 and 2, the user can simply put two fingers on the first electrode set 110 and the second electrode set 120 with the optical sensor 126, respectively, to obtain at least two physiological features simultaneously. Therefore, the biosensor configuration is convenient to the user to obtain many cardiac health statues or other body related information.

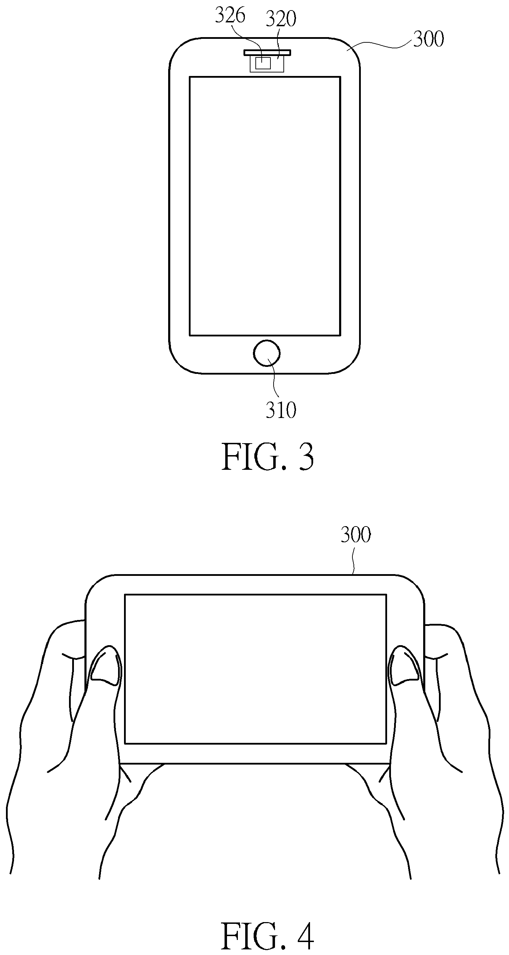

FIG. 3 is a diagram illustrating an electronic device 300 according to a second embodiment of the present invention. The electronic device 300 comprises a first electrode set 310, a second electrode set 320 with an optical sensor 326. In this embodiment, the electronic device 300 is a smartphone, the first electrode set 310 is built with a home button of the smartphone, and the second electrode set 320 with the optical sensor 326 is positioned at a face of the smartphone. In addition, the designs of the second electrode set 320 with the optical sensor 326 is similar to the second electrode set 120 with the optical sensor 126 shown in FIG. 1, that is the electrodes of the second electrode set 320 may be at a peripheral zone of the optical sensor 326, and the second electrode set 320 may have any suitable shape such as the U-shaped or O-shaped electrode.

In the operations of the electronic device 300, the first electrode set 310 is arranged to contact a first position of a human body, and the second electrode set 320 with the optical sensor 326 is arranged contact a second position of the human body, to receive a plurality of physiological signals to determine at least two physiological features (e.g. cardiac health statues) simultaneously. Taking FIG. 4 as an example, a user may put a left thumb on the second electrode set 320 with the optical sensor 326, and put a right thumb on the first electrode set 310. In the embodiment shown in FIG. 3 and FIG. 4, the first electrode set 310 and the second electrode set 320 are arranged to provide physiological signals, and a processor (not shown) within the electronic device 300 generates the ECG signal (e.g. lead I ECG signal) according to the physiological signals from the first electrode set 310 and the second electrode set 320; and the optical sensor 326 may comprise an light emitter and a photodiode, the light emitter generates light to the left thumb of the user, and the photodiode receives the reflected light to generate a physiological signal, and the processor within the electronic device 300 generates the PPG signal according to the physiological signal from the optical sensor. By analyzing the ECG signal and the PPG signal, e.g. analyzing the waveforms and periods of the ECG signal and the PPG signal, the at least two physiological features can be obtained simultaneously, and the physiological features can be displayed on a screen of the electronic device 300 for the reference to the user. For example, the at least two physiological features may comprise cardiac health statues like ECG, heart rate, blood oxygen saturation (SpO2), blood pressure, or other information like bio-impedance, body fat, body muscle, temperature, blood content analysis, or tissue content concentrations (St02, collagen, melanin, water...etc).

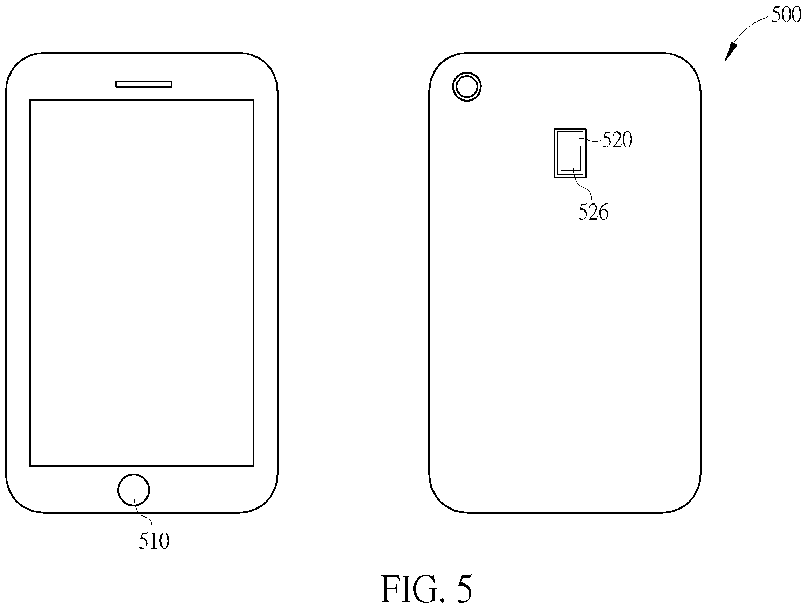

FIG. 5 is a diagram illustrating an electronic device 500 according to a third embodiment of the present invention. The electronic device 500 comprises a first electrode set 510, a second electrode set 520 with an optical sensor 526. In this embodiment, the electronic device 500 is a smartphone, the first electrode set 510 is built with a home button of the smartphone, and the second electrode set 520 with the optical sensor 526 is positioned at a back case of the smartphone. In addition, the designs of the second electrode set 520 with the optical sensor 526 is similar to the second electrode set 120 with the optical sensor 126 shown in FIG. 1, that is the electrodes of the second electrode set 520 may be at a peripheral zone of the optical sensor 526, and the second electrode set 520 may have any suitable shape such as have the U-shaped or O-shaped electrode.

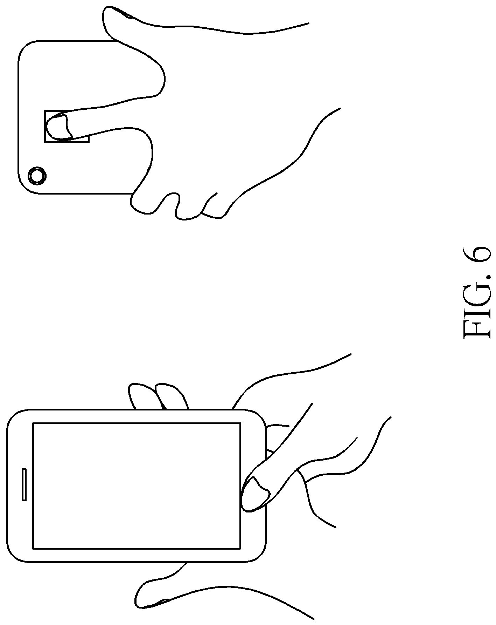

In the operations of the electronic device 500, the first electrode set 510 is arranged to contact a first position of a human body, and the second electrode set 520 with the optical sensor 526 is arranged contact a second position of the human body, to receive a plurality of physiological signals to determine at least two physiological features simultaneously. Taking FIG. 6 as an example, a user may put the left index finger on the second electrode set 520 with the optical sensor 526, and put the right thumb on the first electrode set 510. In the embodiment shown in FIG. 5 and FIG. 6, the first electrode set 510 and the second electrode set 520 are arranged to provide physiological signals, and a processor (not shown) within the electronic device 500 generates the ECG signal (e.g. lead I ECG signal) according to the physiological signals from the first electrode set 510 and the second electrode set 520; and the optical sensor 526 may comprise an light emitter and a photodiode, the light emitter generates light to the left index finger of the user, and the photodiode receives the reflected light to generate a physiological signal, and the processor within the electronic device 500 generates the PPG signal according to the physiological signal from the optical sensor. By analyzing the ECG signal and the PPG signal, e.g. analyzing the waveforms and periods of the ECG signal and the PPG signal, the at least two physiological features can be obtained simultaneously, and the physiological features can be displayed on a screen of the electronic device 500 for the reference to the user. For example, the at least two physiological features may comprise cardiac health statues like ECG, heart rate, blood oxygen saturation (SpO2), blood pressure, or other information like bio-impedance, body fat, body muscle, temperature, blood content analysis, or tissue content concentrations (St02, collagen, melanin, water...etc).

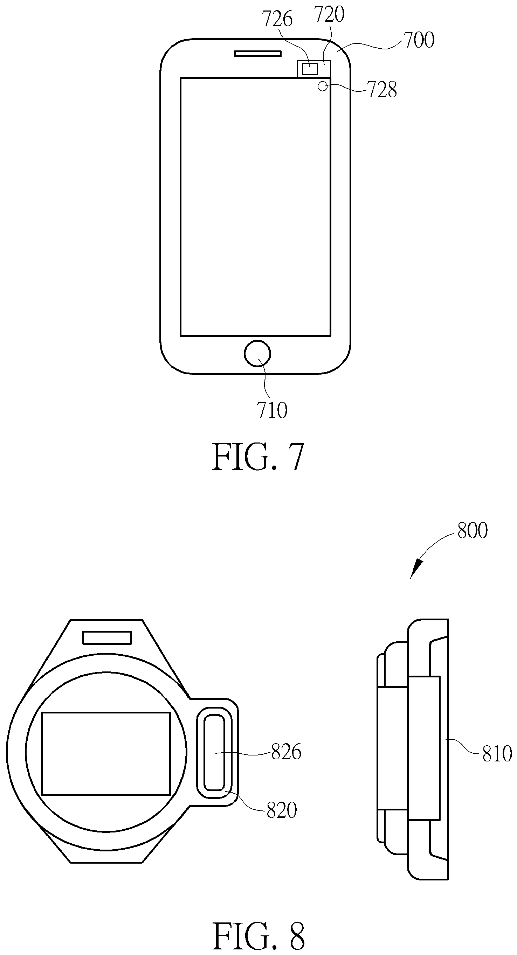

FIG. 7 is a diagram illustrating an electronic device 700 according to a fourth embodiment of the present invention. The electronic device 700 comprises a first electrode set 710, a second electrode set 720 with an optical sensor 726. In this embodiment, the electronic device 700 is a smartphone, the first electrode set 710 is built with a home button of the smartphone, and the second electrode set 720 with the optical sensor 726 is positioned at a face of the smartphone. In addition, the designs of the second electrode set 720 with the optical sensor 726 is similar to the second electrode set 120 with the optical sensor 126 shown in FIG. 1, that is, the electrodes of the second electrode set 720 may be at a peripheral zone of the optical sensor 726, and the second electrode set 720 may have any suitable shape such as the U-shaped or O-shaped electrode.

In the operations of the electronic device 700, the first electrode set 710 is arranged to contact a first position of a human body, and the second electrode set 720 with the optical sensor 726 is arranged contact a second position of the human body, to receive a plurality of physiological signals to determine at least two physiological features simultaneously. Similar to the example shown in FIG. 4, a user may put the left thumb on the second electrode set 720 with the optical sensor 726, and put the right thumb on the first electrode set 710. In the embodiment shown in FIG. 7, the first electrode set 710 and the second electrode set 720 are arranged to provide physiological signals, and a processor (not shown) within the electronic device 700 generates the ECG signal (e.g. lead I ECG signal) according to the physiological signals from the first electrode set 710 and the second electrode set 720; and the optical sensor 726 may comprise a photodiode, and an light-emitted diode 728 built in the screen may generate light to the left thumb of the user, and the photodiode receives the reflected light to generate a physiological signal, and the processor within the electronic device 700 generates the PPG signal according to the physiological signal from the optical sensor. By analyzing the ECG signal and the PPG signal, e.g. analyzing the waveforms and periods of the ECG signal and the PPG signal, the at least two physiological features can be obtained simultaneously, and the physiological features can be displayed on a screen of the electronic device 700 for the reference to the user. For example, the at least two physiological features may comprise cardiac health statues like ECG, heart rate, blood oxygen saturation (SpO2), blood pressure, or other information like bio-impedance, body fat, body muscle, temperature, blood content analysis, or tissue content concentrations (St02, collagen, melanin, water...etc).

FIG. 8 is a diagram illustrating an electronic device 800 according to a fifth embodiment of the present invention. The electronic device 800 comprises a first electrode set 810, a second electrode set 820 with an optical sensor 826. In this embodiment, the electronic device 800 is a watch, the first electrode set 810 is built in the inner side of the watch, and the optical sensor 826 is within the second electrode set 820.

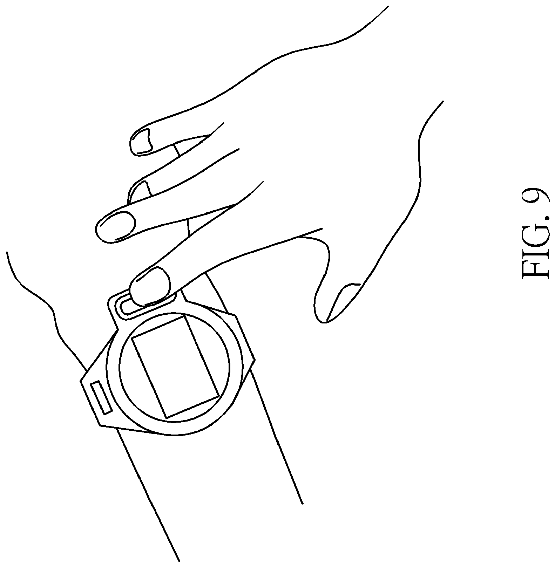

In the operations of the electronic device 800, the first electrode set 810 is arranged to contact a first position of a human body, and the second electrode set 820 with the optical sensor 826 is arranged contact a second position of the human body, to receive a plurality of physiological signals to determine at least two physiological features simultaneously. Taking FIG. 9 as an example, a user may put the right index finger on the second electrode set 820 with the optical sensor 826. In the embodiment shown in FIG. 8 and FIG. 9, the first electrode set 810 and the second electrode set 820 are arranged to provide physiological signals, and a processor (not shown) within the electronic device 800 generates the ECG signal according to the physiological signals from the first electrode set 810 and the second electrode set 820; and the optical sensor 826 may comprise a light emitter and a photodiode, the light emitter generates light to the left index finger of the user, and the photodiode receives the reflected light to generate a physiological signal, and the processor within the electronic device 800 generates the PPG signal according to the physiological signal from the optical sensor. By analyzing the ECG signal and the PPG signal, e.g. analyzing the waveforms and periods of the ECG signal and the PPG signal, the at least two physiological features can be obtained simultaneously, and the physiological features can be displayed on a screen of the electronic device 800 for the reference to the user. For example, the at least two physiological features may comprise cardiac health statues like ECG, heart rate, blood oxygen saturation (Sp02), blood pressure, or other information like bio-impedance, body fat, body muscle, temperature, blood content analysis, or tissue content concentrations (St02, collagen, melanin, water...etc).

Briefly summarized, in the biosensor configuration of the present invention, an optical sensor is built with one electrode set so that the user can simply put two fingers (or any two body positions) on the electronic device to obtain at least two physiological features simultaneously. Therefore, the biosensor configuration is convenient to the user to obtain many physiological features.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. An electronic device (100, 300, 500, 700, 800), comprising:
a first electrode set (110, 310, 510, 710, 810) comprising at least one electrode;
a second electrode set (120, 320, 520, 720, 820) with an optical sensor, wherein the second electrode set comprises at least one electrode ;
wherein the first electrode set (110, 310, 510, 710, 810) is arranged to contact a first position of a human body, and the second electrode set with the optical sensor is arranged contact a second position of the human body, to receive a plurality of physiological signals.

2. The electronic device (100, 300, 500, 700, 800) of claim 1, wherein the second electrode set (120, 320, 520, 720, 820) is at a peripheral zone of the optical sensor.

3. The electronic device (100, 300, 500, 700, 800) of claim 2, wherein the optical sensor is within the second electrode.

4. The electronic device (100, 300, 500, 700, 800) of claim 1, wherein the first electrode set (110, 310, 510, 710, 810) is arranged to contact one of a right half body and a left half body of the human body, and the second electrode set (120, 320, 520, 720, 820) with the optical sensor is arranged to contact the other one of the right half body and the left half body of the human body.

5. The electronic device (100, 300, 500, 700, 800) of claim 4, wherein the first electrode set (110, 310, 510, 710, 810) is arranged to contact one of a right hand and a left hand, and the second electrode set (120, 320, 520, 720, 820) with the optical sensor is arranged to contact the other one of the right hand and the left hand.

6. The electronic device (100, 300, 500, 700, 800) of claim 5, wherein the first electrode set (110, 310, 510, 710, 810) is arranged to contact a finger or thumb of one of the right hand and the left hand, and the second electrode set (120, 320, 520, 720, 820) with the optical sensor is arranged to contact a finger or thumb of the other one of the right hand and the left hand.

7. The electronic device (100, 300, 500, 700, 800) of claim 4, further comprising:
a processor, coupled to the first electrode set (110, 310, 510, 710, 810) and the second electrode set (120, 320, 520, 720, 820) with the optical sensor, for generating an electrocardiography, ECG, signal according to the physiological signals from the first electrode set (110, 310, 510, 710, 810) and the second electrode set (120, 320, 520, 720, 820), and generating an photoplethysmography, PPG, signal according to the physiological signal from the optical sensor.

8. The electronic device (100, 300, 500, 700, 800) of claim 7, wherein the processor analyzing the ECG signal and the PPG signal to obtain at least two physiological features simultaneously.

9. The electronic device (100, 300, 500, 700, 800) of claim 8, wherein the at least two physiological feature comprises ECG, heart rate, blood oxygen saturation, Sp02, blood pressure, bio-impedance, body fat, body muscle, temperature, blood content analysis, or tissue content concentration.

10. The electronic device (100, 300, 500, 700, 800) of claim 1, wherein the electronic device is a smartphone, a pad, a tablet, a watch, an accessary, a wearable device or a patch.

11. The electronic device (100, 300, 500, 700, 800) of claim 1, wherein the electronic device (100, 300, 500, 700, 800) is a smartphone, (i) the first electrode set (110, 310, 510, 710, 810) is positioned at a side of the smartphone, and the second electrode set (120, 320, 520, 720, 820) with the optical sensor are positioned at a back case of the electronic device, or (ii) the first electrode set (110, 310, 510, 710, 810) is positioned at a face of the smartphone, and the second electrode set (120, 320, 520, 720, 820) with the optical sensor are positioned at a back case of the electronic device, or (iii) the electronic device (100, 300, 500, 700, 800) is a smartphone, and the first electrode set (110, 310, 510, 710, 810) and the second electrode set (120, 320, 520, 720, 820) with the optical sensor are positioned at a face of the smartphone, or (iv) the first electrode set (110, 310, 510, 710, 810) and the second electrode set (120, 320, 520, 720, 820) with the optical sensor are positioned at a back case of the smartphone.

12. A method for generating at least two bio-signal or physiological feature, comprising:
using a first electrode set (110, 310, 510, 710, 810) to receive a first physiological signal corresponding a first position of a human body;
using a second electrode set (120, 320, 520, 720, 820) to receive a second physiological signal corresponding a second position of the human body;
using an optical sensor to receive a third physiological signal; and
determining the at least two physiological features according to the first physiological signal, the second physiological signal and the third physiological signal.

13. The method of claim 12, wherein the first position is one of a right half body and a left half body of the human body, and the second position is the other one of the right half body and the left half body of the human body.

14. The method of claim 12, wherein the step of determining the at least two physiological features according to the first physiological signal, the second physiological signal and the third physiological signal comprises:
generating an electrocardiography, ECG, signal according to the first physiological signal and the second physiological signal;
generating an photoplethysmography, PPG, signal according to the third physiological signal; and
analyzing the ECG signal and the PPG signal to obtain the at least two physiological features simultaneously

15. The electronic device of claim 14, wherein the at least two physiological features comprises ECG, heart rate, blood oxygen saturation, Sp02, blood pressure, body fat, body muscle, temperature, blood content analysis, or tissue content concentration bio-impedance.
